# EUROPÄISCHE PATENTSCHRIFT

(11) **EP 0 569 805 B1**
(45) Veröffentlichungstag und Bekanntmachung des Hinweises auf die Patenterteilung: **12.03.1997**
(21) Anmeldenummer: 93107119.5
(22) Anmeldetag: 03.05.1993
(51) Int. Cl.: C07C 69/145, C07C 43/303, C07C 67/08, C07C 29/14, C07C 41/56, C07C 45/42, A01N 37/02

(54) **Verfahren und Zwischenprodukte zur Herstellung von 2,13-Octadecadienylacetat sowie dessen Verwendung**
Process and intermediates for the preparation of 2,13-octadienyle acetate and its use
Procédé et intermédiaires pour la préparation de l'acétate de 2,13-octadiènyle et son utilisation

(30) Priorität: 13.05.1992 DE 4215747
(43) Veröffentlichungstag der Anmeldung: 18.11.1993
(73) Patentinhaber: BASF Aktiengesellschaft, 67063 Ludwigshafen (DE)
(72) Erfinder: Buschmann, Ernst, Dr., W-6700 Ludwigshafen (DE); Klein, Ulrich, Dr., W-6703 Limburgerhof (DE); Mackenroth, Christiane, Dr., W-6702 Bad Duerkheim (DE); Neumann, Ulrich, Dr., W-6707 Schifferstadt (DE); Renz, Guenter, Dr., W-6800 Mannheim 31 (DE); Audemard, Henri Philippe, Dr., F-8400 Avignon (FR)

(56) Entgegenhaltungen:
- PATENT ABSTRACTS OF JAPAN, vol. 17, no. 171 (C-1044), 1993; & JP-A-4 330 032
- CHEMICAL ABSTRACTS, vol. 112, 1990, Columbus, Ohio, US; abstract no. 74266u

## Beschreibung

Die vorliegende Erfindung betrifft ein Verfahren zur Herstellung eines Gemisches aus E2,Z13-Octadecadienylacetat Ia und Z2,Z13-Octadecadienylacetat Ib 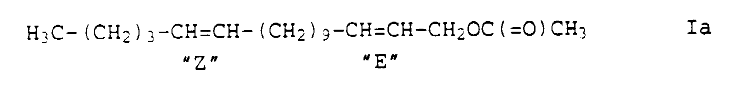 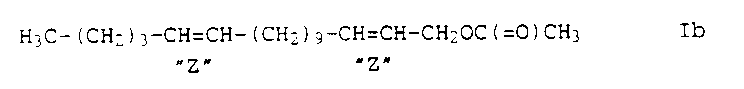 welches dadurch gekennzeichnet ist, daß man Z11-Hexadecenal II 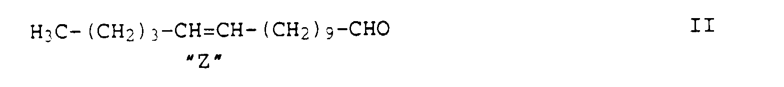 in an sich bekannter Weise in Gegenwart einer Base mit einem Phosphoniumsalz der Formel III

(C₆H₅)₃P⁺CH₂CH(OR¹)OR² X⁻ III

in der R¹ und R² für Alkyl mit 1 bis 8 C-Atomen stehen oder gemeinsam -CH₂CH₂- oder -CH₂CH₂CH₂- bedeuten, wobei in diesen Ketten ein bis drei Wasserstoffatome durch C₁-C₃-Alkylgruppen ersetzt sein können, und X Halogen bedeutet, zum E2,Z13-Octadecadienylacetal IVa bzw. Z2,Z13-Octadecadienylacetal IVb 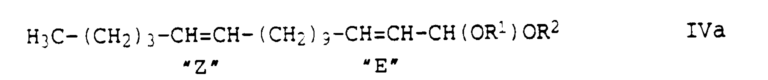 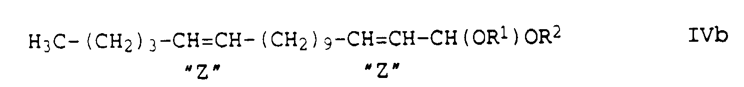 oder unter gleichzeitiger Spaltung des Acetals zum E2,Z13-Octadecadienal Va bzw. Z2,Z13-Octadecadienal Vb 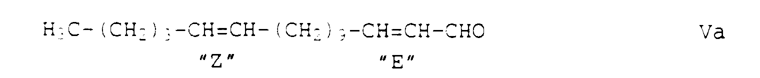 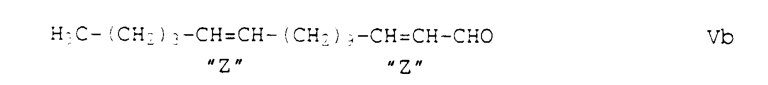 umsetzt, anschließend IVa und IVb bzw. Va und Vb zum entsprechenden Alkohol VIa bzw. VIb 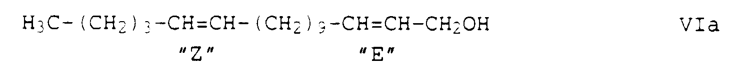 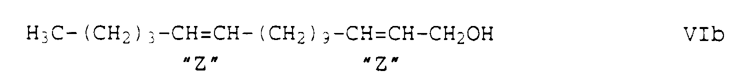 reduziert und VIa und VIb in an sich bekannter Weise acetyliert.

Desweiteren betrifft die Erfindung E2,Z13-Octadecadienylacetale und Z2,Z13-Octadecadienylacetale der Formeln IVa und IVb 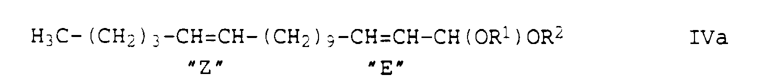 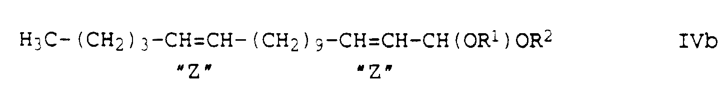 in denen R¹ und R² für Alkyl mit 1 bis 8 C-Atomen stehen oder gemeinsam -CH₂CH₂- oder -CH₂CH₂CH₂- bedeuten, wobei in diesen Ketten ein bis drei Wasserstoffatome durch C₁-C₃-Alkylgruppen ersetzt sein können und deren Verwendung als schenprodukte.

Die Erfindung betrifft außerdem Mittel und Verfahren zur Bekämpfung der Leopardenmotte (Zeuzera pyrina) mittels eines Gemisches aus E2,Z13-Octadecadienylacetat Ia und Z2,Z13-Octadecadienylacetat Ib.

Aus der Literatur ist E2,Z13-Octadecadienylacetat Ia als Pheromon von Schädlingen der Ordnung der Lepidopteren bekannt [Frerot et al., C. R. Acad. Sc. Paris, t. 302, Serie II, n^{o} 7, 413 (1986); Tonini et al., J. Chem. Ecol., 12(6), 1545 (1986); Meyer Schwarz et al., Tetrahedron Lett., 24(10), 1007 (1983)].

Außerdem ist aus der Literatur bekannt, daß E2,Z13-Octadecadienylacetat Ia bei Schmetterlingen der Arten Vitacea polistiformis, Synanthedon tupiliformis und Monopsis rusticella als Lockstoff wirkt [Meyer Schwarz et al., Tetrahedron Lett., 24(10), 1007 (1983); Szocs et al., Entomol. Exp. Appl. 39(2), 131 (1985); Voerman et al., J. Chem. Ecol. 10(9), 1371 (1984); Szocs et al., Biochem. Syst. Ecol., 17(5), 417 (1983)], wohingegen E2,Z13-Octadecadienylacetat Ia gegen die Leopardenmotte (Zeuzera pyrina) keine derartige Wirkung entwickelt [Tonini et al., J. Chem. Ecol., 12(6), 1552 (1986)].

Die Leopardenmotte Zeuzera pyrina ist ein Holzbohrer mit weltweiter Verbreitung. In Süd- und Westeuropa befällt die Larve Birnen-, Äpfel- und Olivenbäume.

Die frisch geschlüpften Larven dringen in die dünneren äußeren Zweige ein. Ältere Larven befallen dickere Zweige und richten verstärkt Schaden an. Die Bekämpfung des Schädlings ist schwierig, da die Larven unter der Baumrinde vor Insektiziden gut geschützt sind. Die Suche galt deshalb einem alternativen Bekämpfungsverfahren.

Es ist bekannt, daß bei Schmetterlingen von paarungsbereiten weiblichen Tieren Sexuallockstoffe (Pheromone) erzeugt und in die Umgebung ausgeschieden werden. Männliche Schmetterlinge derselben Art können dann mit Hilfe dieses Riechstoffes die Weibchen auffinden.

Grundsätzlich gibt es drei verschiedene Möglichkeiten, Sexuallockstoffe (Pheromone) von Schmetterlingen im Pflanzenschutz anzuwenden:

### 1. Monitortechnik

Sogenannte Pheromonfallen, bestückt mit synthetischen Sexuallockstoffködern werden in den Befallsgebieten ausgehängt. Der Fallenfang von männlichen Faltern erbringt den Nachweis des Auftretens des Schädlings. Außerdem lassen sich Hinweise zur Befallsstarke und auf den richtigen Zeitpunkt der Bekämpfung ableiten.

### 2. Abfangtechnik

Man kann den Lockstoff mit insektiziden Wirkstoffen kombinieren. Es besteht die Möglichkeit, dem Köder oder der Falle, Insektizide zuzusetzen oder aber die unmittelbare Umgebung der Falle zu behandeln. Damit kann dann der größte Teil, der aus weiter Entfernung angelockten männlichen Falterbevölkerung abgetötet werden.

### 3. Paarungsstörungstechnik

Durch geeignete Dispenser stellt man eine gleichmäßige Verteilung des Sexualpheromons im Befallsgebiet sicher. Die männlichen Schmetterlinge werden dadurch am Auffinden der Weibchen gestört. Die Paarung unterbleibt.Der Schädlingsbefall geht zurück.

Zahlreiche Sexualpheromone von Schmetterlingen wurden in der Vergangenheit isoliert und für das Monitoring als hochwirksame Lockstoffe eingesetzt. Nur wenige dieser Lockstoffe lassen sich für die Paarungsstörungsmethode verwenden. M. Kehat et al. (Phytoparasitica 13, 215 - 220 (1985)) erzielten sehr schlechte Ergebnisse bei Versuchen zur Paarungsstörung mit dem Pheromon von Spodoptera littoralis. C. P. Whittle und T. E. Bellas (Chemistry in Australia 198 - 201 (1988)) verweisen darauf, daß die Gründe für den vielfachen Mißerfolg der Paarungsstörungsmethode wissenschaftlich nicht begründbar sind. J.Möhl (Beitr. Ent., Berlin, 200ff, 1985) berichtet, daß bei mehr als 20 Schmetterlingsarten versucht wurde, die Paarung mit Sexualpheromonen zu stören. Es konnten jedoch nur bei einzelnen Arten Erfolge erzielt werden. Vergebliche Versuche mit dem Pheromon von Adoxophyesarten berichtet Y.Tamaki (J. Appl. Entomol. Zool. 27 124 - 130 (1983)).

Eine neue Monographie zum Thema: Insektenpheromone im Pflanzenschutz, J. Wiley and Sons, 1989, Herausgeber A. R. Jutsum, R. F. S. Gordon, berichtet von zahlreichen Fehlschlagen z. B. D. G. Campion auf S. 92: "Obwohl viele Pheromone jetzt isoliert worden sind, wird nur über wenige erfolgreiche Paarungsstörungsversuche berichtet. Eine Ausnahme ist der Pink bollworm." S. 113:"Kontrolle des bekreuzten Traubenwicklers war in einigen Gebieten erfolgreich in anderen nicht." S. 114: "Bei Heliothisarten kein Erfolg oder unklare Ergebnisse; bei Spodopteraarten war die Paarungsstörungstechnik schwierig."

C. S. Sanders auf S. 328: "Mit hunderten von Sexualpheromonen wurde die Paarungsstörungstechnik erprobt. Die Zahl der Erfolge ist sehr begrenzt."

Als Grund für die vielen Fehlschläge wird auf S. 340 - 341 das mangelnde Verstehen der biologischen Zusammenhänge angesehen.

Nach W. Roelofs (J. Chem. Ecol. 4, 685 - 698 (1978)) sind diejenigen Substanzen für die Paarungsstörung gut geeignet, die optimale Lockwirkung besitzen.

Voraussetzung für die Durchführung der Paarungsstörungsmethode ist die Verfügbarkeit von größeren Mengen Pheromonwirkstoff. Die bisher publizierten Verfahren zur Herstellung benötigen zahlreiche Rekationsschritte und sind deshalb für die Herstellung von Kilogramm-Mengen ungeeignet, selbst wenn man wirtschaftliche Erwägungen nicht berücksichtigt.

Als Beispiel für derartige akademische Synthesen seien genannt die Methode von B. Frerot et al. C. R. Acad. Sc. Paris 302 II, (7) 413 - 415 (1986).

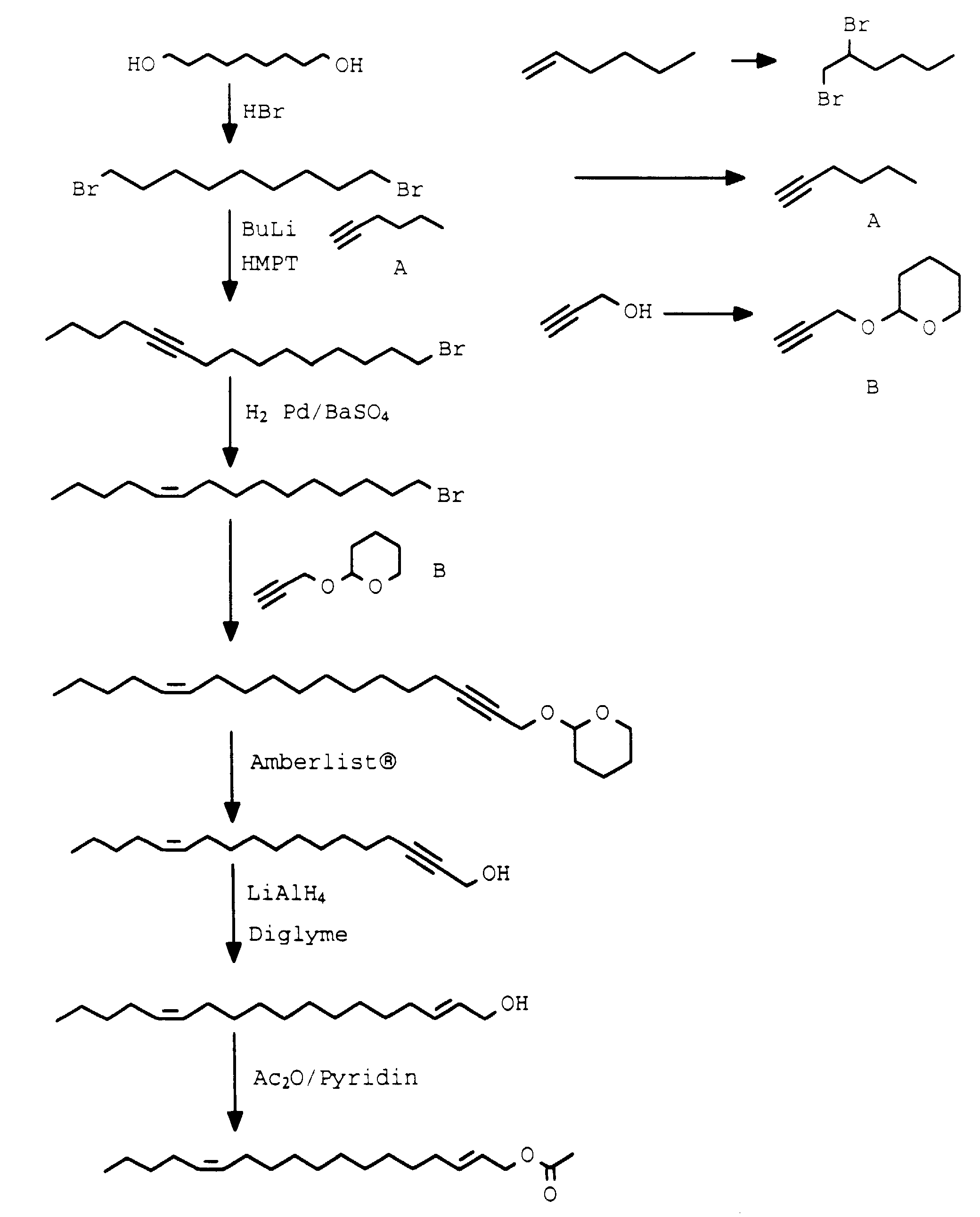

Es handelt sich um eine siebenstufige Synthese. Die sechste Stufe nutzt LiAlH₄, ein Reduktionsmittel mit hohem Gefahrenpotential, das nur in Spezialapparaturen eingesetzt werden kann. Zur Herstellung von Vorprodukten (A, B) sind weitere drei Verfahrensschritte erforderlich.

Ein alternatives Synthesekonzept findet sich bei S. Voerman et al. Journal of Chem. Ecol. 10,(9) 1371 - 1376 (1984):

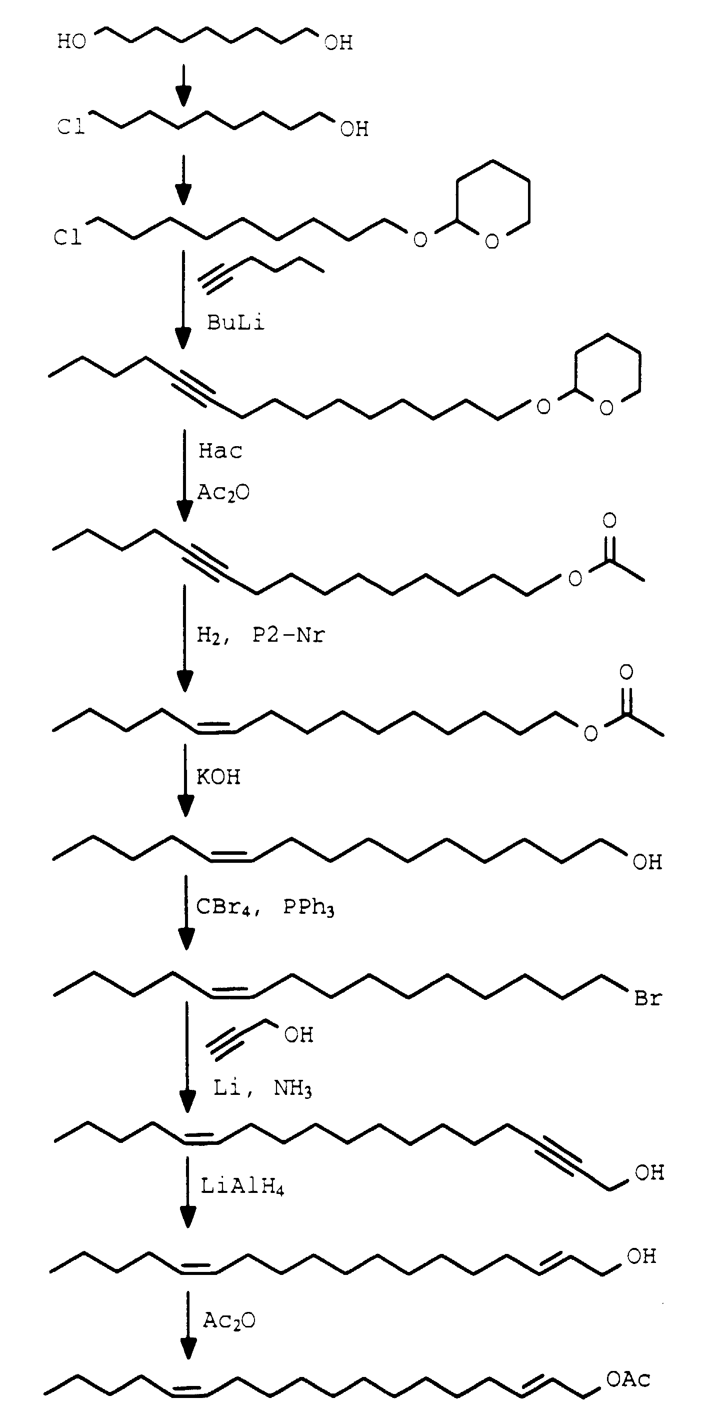

Auch diese zehnstufige Synthese benötigt LiAlH₄. Stufe 8 erfordert eine weitere Spezialapparatur zur Umsetzung in flüssigem Ammoniak.

Weitere Herstellverfahren für E2,Z13-Octadecadienylacetat wurden veröffentlicht:
M. Hoskovec et al., Coll. czech. Chem. Commun. 55 (9) 2270 - 81 (1990),
M. Schwarz et al. Tetrahedron Letters 24 (10) 1007 - 1010 (1983)
B. G. Kovalev et al. Khim. Prir. Soedin. (2) 264 - 6 (1989)
C. Descoins et al., Synth. Commun. 19 (15) 2703 - 2712 (1989)
M. L. Sharma et al., Indian J. Chem. Sect. B, 29 (7), 657-658 (1990)
C. Tonini et al., J. Chem. Ecol. 12 (6) 1545 - 1558 (1986)
A. Yamamoto, European Patent Application 419 330.

Der vorliegenden Erfindung lag ein ökonomisches Verfahren zur Bekämpfung der Leopardenmotte und die Bereitstellung der dafür erforderlichen Mittel als Aufgabe zugrunde.

Demgemäß wurde das eingangs geschilderte Verfahren zur Herstellung eines Gemisches aus E2,Z13-Octadecadienylacetat Ia und Z2,Z13-Octadecadienylacetat Ib ausgehend von Z11-Hexadecanal II und einem Phosphoniumsalz der Formel III und neue Zwischenprodukte IVa und IVb gefunden.

Desweiteren wurde gefunden, daß sich Leopardenmotten mittels der Verwirrungsmethode durch E2,Z13-Octadecadienylacetat Ia oder durch Gemische aus E2,Z13-Octadecadienylacetat Ia und Z2,Z13-Octadecadienylacetat Ib bekämpfen lassen.

Das Verfahren zur Herstellung des Gemisches aus E2,Z13-Octadecadienylacetat Ia und Z2,Z13-Octadecadienylacetat Ib wird im einzelnen wie folgt durchgeführt:

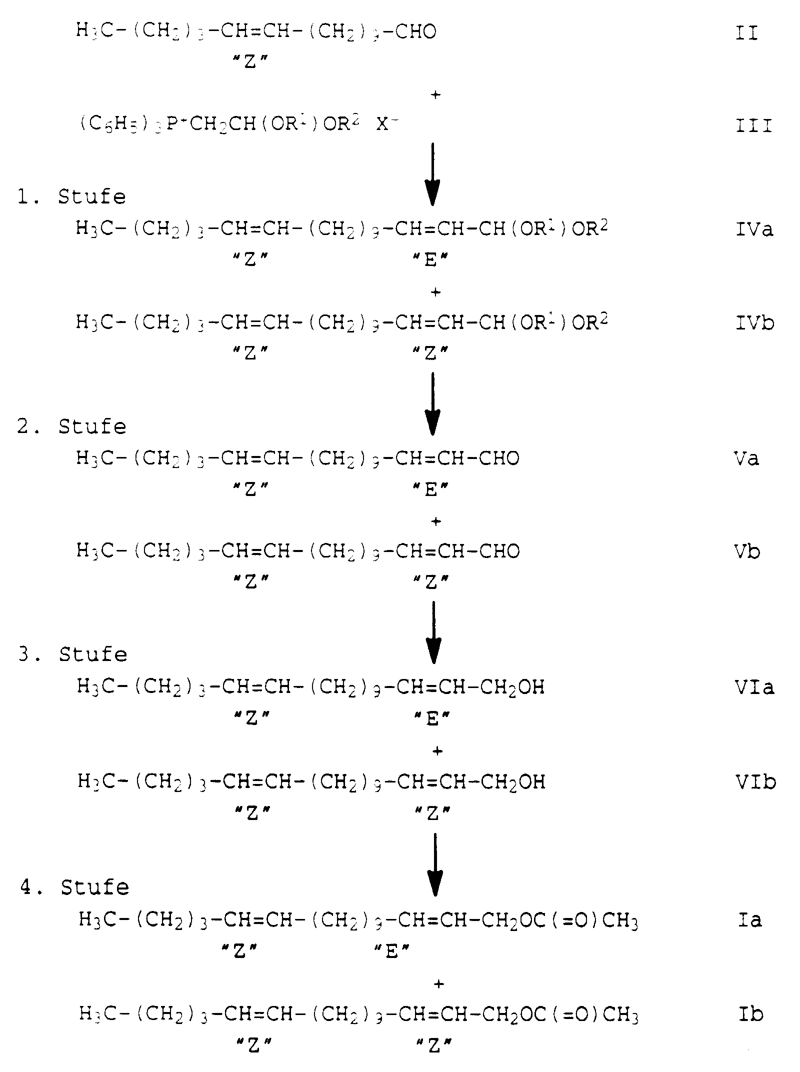

Die Reste R¹ und R² in den Formeln III und IVa bzw. IVb stehen für Alkyl mit 1 bis 8 C-Atomen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise C₁-C₄-Alkyl; oder R¹ und R² stehen gemeinsam für -CH₂CH₂- oder -CH₂CH₂CH₂-, wobei in diesen Ketten ein bis drei Wasserstoffatome durch C₁-C₃-Alkylgruppen wie vorstehend genannt ersetzt sein können.

X in der Formel III bedeutet ein Halogenatom wie insbesondere Chlor, Brom und Jod.

Die Verfahrensschritte werden im einzelnen wie folgt durchgeführt:

### 1. Stufe: Wittig-Reaktion

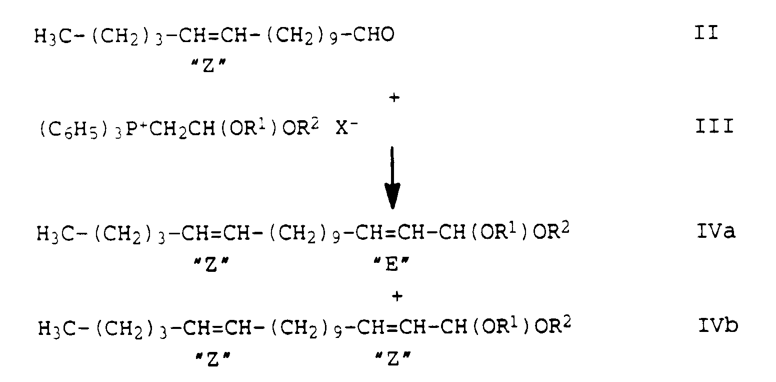

Diese Umsetzung wird üblicherweise bei Temperaturen von -20°C bis 100°C, vorzugsweise 0°C bis 50°C in Gegenwart einer Base durchgeführt.

Insbesondere geeignete Lösungsmittel sind z.B. am Stickstoff vollständig sübstituierte Säureamide wie Dimethylformamid, Dimethylacetamid, N-Methylpyrrolidon, Sulfone wie Dimethylsulfon, Sulfoxide wie Dimethylsulfoxid, Ether wie Tetrahydrofuran, Glykolether wie Dimethoxyethan, Diethoxyethan, Diglyme, aromatische Kohlenwasserstoffe wie Benzol oder Toluol, halogenierte Kohlenwasserstoffe wie Methylenchlorid oder Chlorbenzol.

Es können auch Gemische der genannten Lösungsmittel verwendet werden.

Als Basen dienen vorzugsweise niedermolekulare Alkoholate, z. B. Alkalialkoholate wie Natriummethylat, Natriumethylat, Kalium-tert.-butylat, sowie Alkalihydroxide bei Verwendung eines Zweiphasensystems. Daneben können Basen, wie Butyllithium, Alkalihydride oder -amide verwendet werden, wobei die Reaktion ggf. unter Schutzgas durchgeführt wird.

Die Basenmenge ist nicht besonders kritisch, üblicherweise werden pro Mol Phosphoniumhalogenid stöchiometrische Mengen der Base oder ein geringer Überschuß z.B. 1 bis 20 Mol-%, verwendet.

Nach Zugabe der Base wird Z11-Hexadecenal in äquimolare Menge oder im 10 - 20 %igem Unterschuß zudosiert.

Je nach Wahl von Reaktionsmedium, Base und Temperatur entstehen unterschiedliche Gemisch der in Position Z oder E konfigurierten Derivate.

Die Acetale VIII können nun isoliert werden oder auch ohne Isolierung mit Wasser unter saurer Katalyse zum E2,Z13-Octadecadienalen Va bzw. Vb umgesetzt werden.

Die für die Herstellung benötigten Phosphoniumsalze III erhält man analog der von Sargent et al. beschriebenen Methode [J. Chem. Soc. PT I, 37 (1974)] wie folgt:

Man erwärmt Triphenylphosphin mit einem Halogenacetaldehydacetal der Formel VIII 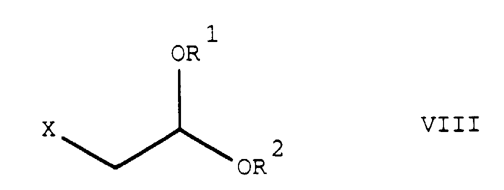 in der X und R¹ und R² die vorstehend angegebene Bedeutung haben in einem geeigneten Lösungsmittel wie Toluol, Xylol, Dimethylformamid, N-Methylpyrrolidon mit oder ohne Druck auf Temperaturen von 80 bis 200°C, vorzugsweise auf 100 - 160°C. Die dabei gebildeten Phosphoniumsalze können als Feststoffe isoliert werden. Oft ist es aber zweckmäßig, die Lösungen oder Suspensionen von III direkt weiter mit einer geeigneten Base umzusetzen.

### 2. Stufe: Acetal-Spaltung

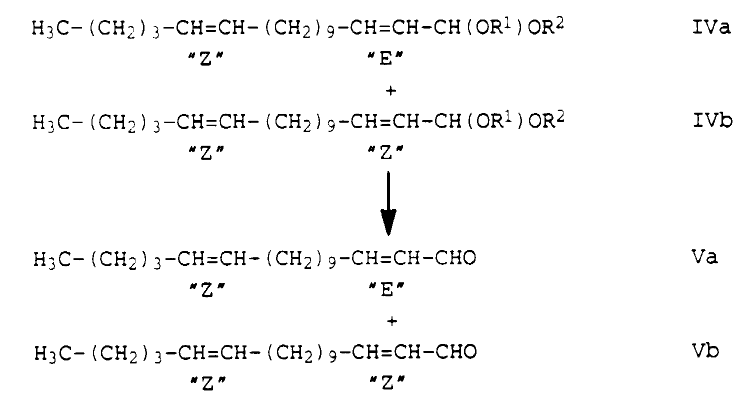

Diese Acetal-Spaltung wird üblicherweise mit mindestens äquivalenten Mengen an Wasser bei Temperaturen von 10°C bis 150°C, vorzugsweise 20°C bis 80°C in Gegenwart einer Säure und gegebenenfalls in Anwesenheit eines zusätzlichen Lösungsmittels durchgeführt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid.

Man verwendet mindestens 1 mol-Äq. Wasser. Bevorzugt wird bei einem Überschuß von 20 bis 100 mol-% oder in Wasser als Lösungsmittel umgesetzt.

Als Säuren können Mineralsäuren oder organische Säuren wie Carbonsäure, Dicarbonsäuren oder Sulfonsäuren verwendet werden. Beispielweise seien Salzsäure, Essigsäure, Oxalsäure oder Toluolsulfonsäure genannt.

Pro Mol Acetal IVa bzw. IVb werden bis zu 2 Säureäguivalente zugesetzt. Vorteilhaft ist auch die Zugabe von Aldehyden wie Propionaldehyd, Benzaldehyd, Butyraldehyd, die den abgespalteten Alkohol übernehmen und somit dem Gleichgewicht entziehen.

### 3. Stufe: Reduktion zum Alkohol

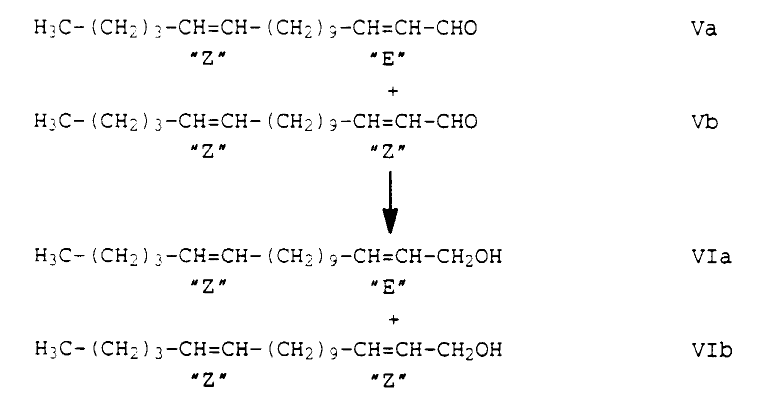

Diese Reduktion wird üblicherweise bei Temperaturen von -10°C bis 150°C, vorzugsweise 0°C bis 50°C durchgeführt.

Geeignete Lösungsmittel sind Ether wie Diethylether, Diisopropylether, tert.-Butylmethylether, Diglyme, Dioxan und Tetrahydrofuran, sowie vorzugsweise Alkohole wie n-Propanol, Isopropanol, n-Butanol und tert.-Butanol, insbesondere Methanol und Ethanol.

Es können auch Gemische der genannten Lösungsmittel verwendet werden.

Als Reduktionsmittel kommen insbesondere Hydride wie Natrium-borhyhrid und Lithium-aluminiumhydrid und Wasserstoff in Gegenwart von üblichen Edelmetallkatalysatoren in Betracht.

Die Reduktionsmittel werden im allgemeinen äquimolar oder im Überschuß verwendet.

### 4. Stufe: Acetylierung

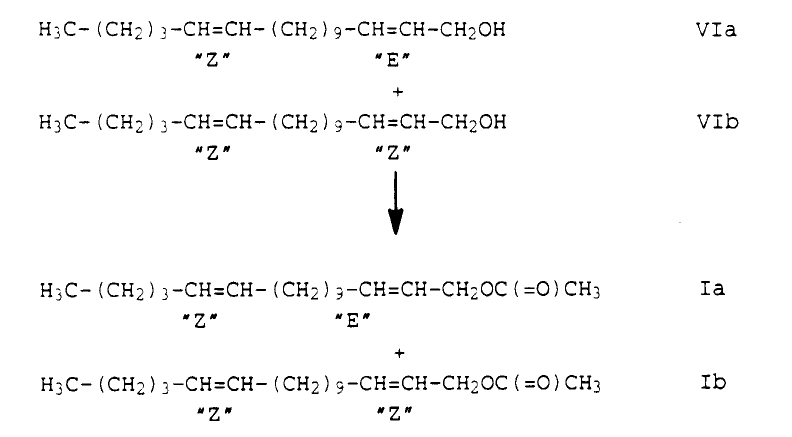

Diese Umsetzung wird üblicherweise bei Temperaturen von -10°C bis 150°C, vorzugsweise 0°C bis 50°C durchgeführt.

Geeignete Acetylierungsmittel sind Acetanhydrid, Acetylchlorid, Acetylbromid oder auch Essigsäure. Die Umsetzung erfolgt mit molaren oder katalytischen Mengen an Base (z. B. Triethylamin, Pyridin, 4-Dimethylaminopyridin) oder auch mit katalytischen Mengen an Säure wie HCl, Essigsäure, Schwefelsäure in einem geeigneten Lösungsmittel wie Kohlenwasserstofffen (Toluol, Xylol, Chlorbenzol, Ligroin), Ethern (Dibutylether, THF), Chlorkohlenwasserstoffen (Chlorbenzol, Methylenchlorid) oder auch lösungsmittelfrei.

Die Acetale IVa und IVb sind neu. Im Hinblick auf ihre Verwendung als Zwischenprodukte zur Synthese der Acetate Ia und Ib sind insbesondere solche Acetale IVa bzw IVb bevorzugt, in denen die Reste R¹ und R² die folgenden Bedeutung haben:

Alkyl mit 1 bis 8 C-Atomen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise C₁-C₄-Alkyl; oder R¹ und R² stehen gemeinsam für -CH₂CH₂-oder -CH₂CH₂CH₂-, wobei in diesen Ketten ein bis drei serstoffatome durch C₁-C₃-Alkylgruppen wie vorstehend genannt ersetzt sein können, insbesondere -CH₂CH₂-, -CH(CH₃)CH₂-, -CH(CH₃)CH(CH₃)-, -CH₂CH₂CH₂-, -CH(CH₃)CH₂CH₂-, -CH₂CH(CH₃)CH₂-, -CH(CH₃)CH(CH₃)CH₂-, -CH(CH₃)CH₂CH(CH₃)-, -CH(CH₃)CH(CH₃)CH(CH₃)- und -CH₂-C(CH₃)₂-CH₂-.

Das nach diesem Verfahren gewonnene Gemisch der Verbindungen Ia und Ib bzw. Ia, Ib, Va und Vb eignet sich zur Bekämpfung der Leopardenmotte (Zeuzera pyrina) insbesondere nach der Paarungsstörungsmethode.

Das Gemisch kann zusammen mit üblichen Hilfsstoffen, z.B. entsprechend präparierten Streifen aus Kunststoff, Bindegarnen, lockstoffgefüllten Ampullen o.ä. (beispielsweise gemäß DE-A 36 40 880 oder DE-A 41 01 878) angewendet werden und auch herstellungsbedingte Verunreinigungen enthalten.

Zur Formulierung des Gemisches kommen sowohl flüssige wie auch feste Präparationen in Frage. Als Lösungsmittel kommen hochsiedende, aromatische, aliphatische oder cycloaliphatische Verbindungen in Betracht. Neben Kohlenwasserstoffen eignen sich Ester, Ether oder Ketone besonders gut. Typische Vertreter dieser Klassen sind z.B. Xylol, Methylnaphthaline, Paraffinöle, Cyclohexanon, Ethylglykolacetat, Isophoron und Dibutylphthalat. Diese Lösungsmittel können allein oder in Mischungen mit anderen Komponenten Verwendung finden. Die den Verbindungen Ia bis Id entsprechenden gesättigten C₁₂-Alkohole und C₁₂-Ester sowie deren Homologe sind besonderes geeignete Formulierungshilfsmittel und können als Synergisten angesehen werden, da sie die Wirkung von Ia, Ib und gegebenenfalls VIa und VIb verstärken.

Weiterhin können Lösungen in pflanzlichen, tierischen oder synthetischen Ölen oder Fetten und anderen verdunstungshemmenden Lösungsmittel mit niedrigem Dampfdruck wie z.B. Dioctylphthalat zum Zwecke der Wirkungsverlängerung hergestellt werden.

Des weiteren ist es möglich, das Gemisch in oder an natürliche oder synthetische feste Träger wie Gummi, Kork, Zellulose, Kunststoffe, gemahlene Kohle, Holzmehl, Silikate, Bimskies, gebrannten Ton oder ähnliche feste Trägerstoffe zu binden oder in speziellen Kapselformulierungen oder Kunststoffbehältern einzusetzen, um so eine gleichmäßige Abgabe an die Luft über längere Zeiträume hinweg zu erreichen. Außerdem kann der Wirkstoff aus geeigneten Behältern, z.B. Kapillaren oder anderen Gefäßen, durch enge Öffnungen oder durch Diffusion durch die Behälterwand sowie aus mehrschichtigen Kunststoffplättchen, sogenannten Flakes, zur Verdunstung gebracht werden, wodurch über längere Zeiträume hinweg besonders gleichmäßige Duftkonzentrationen erzielt werden.

Der Gehalt dieser Zubereitungen an Gemisch kann innerhalb weiter Grenzen schwanken. Generell kann das Verhältnis Wirkstoff:Zusatzstoff z.B. im Bereich von 10:1 bis 1:10³ liegen. In Kapselformulierungen oder anderen geeigneten Behältern kann z.B. der Wirkstoff in reiner, unverdünnter Form angewendet werden und sein Gewichtsanteil, bezogen auf die samt formulierung, sehr hoch sein und bis zu 90 % betragen. Im allgemeinen genügen jedoch sehr geringe Wirkstoffkonzentrationen in den Zübereitungen, um die gewünschte Wirkung auf Leopardenmotten-Männchen auszuüben. Bevorzugt ist ein Mengenverhältnis Wirkstoff :Zusatzstoff von 1:3 bis 1:10³ insbesondere 1:10 bis 1:100.

### Herstellungsbeispiele

### Beispiel 1

### 2-Brommethyl-5,5-dimethyl-1,3-dioxan

Eine Mischung von 1970 g (10 Mol) Bromacetaldehyddiethylacetal und 1040 g (10 Mol) Neopentylglykol wird erwärmt. Bei 96°C beginnt Ethanol abzudestillieren. Die Innentemperatur wird allmählich auf 150°C erhöht, bis kein Ethanol mehr überdestilliert. Man kühlt auf 120°C ab, legt bei dieser Temperatur Wasserstrahlvakuum an, das allmählich bis auf 40 mbar gesteigert wird. Dabei wird wieder Ethanol abdestilliert. Die Übergangstemperatur steigt allmählich bis auf 100°C an. Der Rückstand besteht zu 97,9 % aus 2-Brommethyl-5,5-dimethyl-1,3-dioxan und kann ,ohne weitere Reinigung in die Folgestufen eingesetzt werden.

### Beispiel 2

### E2,Z13-Octadecadienal

Eine Lösung von 288,2 g (1,1 Mol) Triphenylphosphin, 237 g (1,1 Mol) 2-Brommethyl-5,5-dimethyl-1,3-dioxan (97 %ig) in 400 ml DMF und 100 ml Xylol wird 20 h zum Rückfluß erwärmt. Man kühlt auf Raumtemperatur ab, gibt 300 ml Xylol und 123,2 g Kalium-tert.-butylat zu. Die Temperatur steigt dabei auf 37°C. Nach 2 h Rühren bei Raumtemperatur wird 238 g (1 Mol) Z11-Hexadecenal zugetropft (Temperaturanstieg dabei auf 64°C). Man rührt 15 h bei Raumtemperatur und gibt 182,5g (0,5 Mol) 10 %ige HCl und 63,8 g (1,1 Mol) Propionaldehyd zu. Nach 3 h Rühren bei 60°C wird auf Raumtemperatur abgekühlt. Die wäßrige Phase wird mit Xylol extrahiert. Die Xylolphase wird mit Wasser und mit wäßriger NaHCO₃Lösung gewaschen. Nach Einengen am Rotationsverdampfer wird mit Essigester/Petrolether (1:5) aufgenommen und auf 0°C abgekühlt. Das ausgefallene Triphenylphosphinoxid wird abgesaugt. Nach Einengen des Filtrates verbleiben 314,2 g E2,Z13-Octadecadienal (Reinheit 74,6 %).

### Beispiel 3

### E2,Z13-Octadecadienol

Zu einer Lösung von 11,2 g (0,30 Mol) NaBH₄ in 200 ml THF und 200 ml H₂O wird 314,2 g (0,89 Mol) E2,Z13-Octadecadienal (74,6 %ig) bei 10 - 20°C zugetropft. Man rührt 15 h bei Raumtemperatur, stellt mit 10 %iger HCl auf pH 5 ein und extrahiert die organische Phase mit Methyl-tert.butylether. Nach Trocknen über Na₂SO₄ wird eingeengt.
Rückstand: 298,3 g (Reinheit 82 %)

### E2,Z13-Octadecadienylacetat

298,3 g (0,92 Mol) E2,Z13-Octadecadienol (82 %ig) werden vorgelegt. Bei 70°C werden 112,6 g (1,1 Mol) Essigsäureanhydrid zugetrofpt. Man rührt 4 h bei 70°C und destilliert die Essigsäure am Rotationsverdampfer ab. Rückstand (326,2g, Reinheit 85 %).

Sambaydestillation führt zu einer weiteren Anreicherung auf 87,8 % (Siedepunkt 200°C 0,7 mbar)

### Durchführung der Versuche:

Der synthetisch hergestellte Pheromonwirkstoff (Zusammen setzung: 85 % E2,Z13-Octadecadienylacetat, 13 % Isomere) wird in kleine Ampullen aus Kunststoff abgefüllt (Beschreibung siehe DE-OS 36 40 880). Abfüllmenge 234 mg pro Ampulle. Der Wirkstoff diffundiert in der Apfelanlage langsam durch die Wand der Ampulle. Bei Durchschnittstemperaturen von 18°C bis 24°C wurde in den behandelten Flächen eine durchschnittliche Wirkstoffabgabe von ca. 0,8 mg pro Tag und Ampulle gemessen. Die Ampullen werden in zwei Zeuzerabefallenen Apfelgebieten ausgebracht.

Parzelle 1: Domaine St. Paul, Montfavet, Frankreich, Apfelsorte Résistante à la Tavelure.

Parzelle 2: Les Vignères, Cavaillon (Vaucluse) Frankreich, Apfelsorte Golden Smoothee und Granny Smith.

Einige Pheromonampullen werden auch in den Randgebieten der Apfelanlage ausgehängt, um Einflug befruchteter Weibchen zu verhindern.

Der Befall der Apfelbäume wird visuell untersucht. In Parzelle 1 wiesen 50 von 276 untersuchten Bäumen (18 %) Befall auf. In Parzelle 2:36 von 540 (6,7 %).

| Parzelle | Fläche | Zahl der Bäume | Zahl der Ampullen | % Befall 1990 | % Befall 1991 |
|---|---|---|---|---|---|
| 1 | 0,5 ha | 276 | 442 | 100 % | 18 % |
| 2 | 1,2 ha | 1188 | 712 | 28% | 6,7 % |

Parzelle 1 war im Vorjahr sehr viel stärker befallen. Die Ampullen wurden deshalb dort etwas dichter aufgehängt. 1991 ist der Befall immer noch höher als in Parzelle 2. Die Pheromonbehandlung war dennoch erfolgreich. Es wurde bewiesen, daß man mit E2,Z13-Octadecadienylacetat den Schädling Zeuzera pyrina erfolgreich bekämpfen kann.

## Patentansprüche

1. Verfahren zur Herstellung eines Gemisches aus E2,Z13-Octadecadienylacetat Ia und Z2,Z13-Octadecadienylacetat Ib 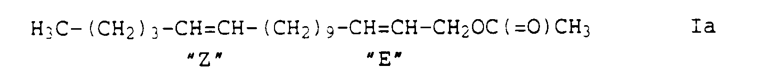 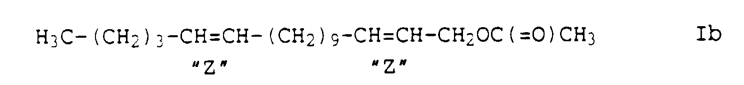 dadurch gekennzeichnet, daß man Z11-Hexadecenal II 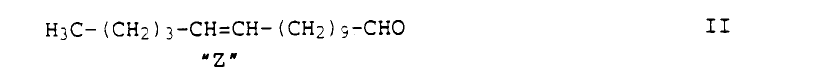 in an sich bekannter Weise in Gegenwart einer Base mit einem Phosphoniumsalz der Formel III
(C₆H₅)₃P⁺CH₂CH(OR¹)OR² X⁻ III
in der R¹ und R² für Alkyl mit 1 bis 8 C-Atomen stehen oder gemeinsam -CH₂CH₂- oder -CH₂CH₂CH₂- bedeuten, wobei in diesen Ketten ein bis drei Wasserstoffatome durch C₁-C₃-Alkylgruppen ersetzt sein können, und X Halogen bedeutet, zum E2,Z13-Octadecadienylacetal IVa bzw. Z2,Z13-Octadecadienylacetal IVb 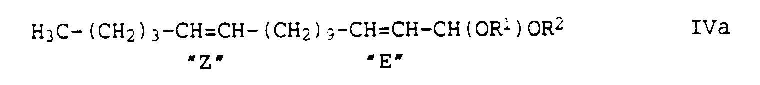 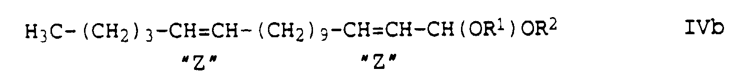 oder unter gleichzeitiger Spaltung des Acetals zum E2,Z13-Octadecadienal Va bzw. Z2,Z13-Octadecadienal Vb 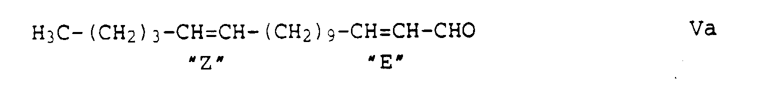 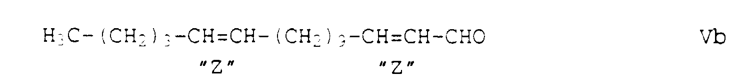 umsetzt, anschließend IVa und IVb bzw. Va und Vb zum entsprechenden Alkohol VIa bzw. VIb 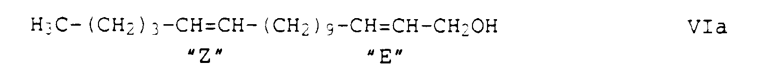 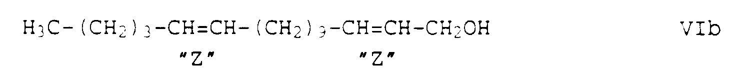 reduziert und VIa und VIb in an sich bekannter Weise acetyliert.

2. E2,Z13-Octadecadienylacetale und Z2,Z13-Octadecadienylacetale der Formeln IVa und IVb 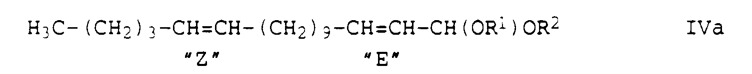 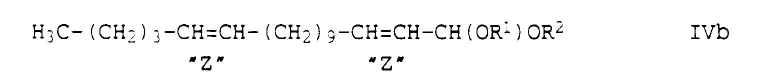 in denen R¹ und R² für Alkyl mit 1 bis 8 C-Atomen stehen oder gemeinsam -CH₂CH₂- oder -CH₂CH₂CH₂- bedeuten, wobei in diesen Ketten ein bis drei Wasserstoffatome durch C₁-C₃-Alkylgruppen ersetzt sein können.

3. Verwendung von E2,Z13-Octadecadienylacetalen und Z2,Z13-Octadecadienylacetalen der Formeln IVa und IVb gemäß Anspruch 2 als Zwischenprodukte.

4. Verwendung von E2,Z13-Octadecadienylacetalen und Z2,Z13-Octadecadienylacetalen der Formeln IVa und IVb gemäß Anspruch 3 zur Herstellung eines Gemisches aus E2,Z13-Octadecadienylacetat Ia und Z2,Z13-Octadecadienylacetat Ib.

5. Verfahren zur Bekämpfung der Leopardenmotte (Zeuzera pyrina) mittels E2,Z13-Octadecadienylacetat Ia.

6. Verfahren zur Bekämpfung der Leopardenmotte (Zeuzera pyrina) mittels eines Gemisches aus E2,Z13-Octadecadienylacetat Ia und Z2,Z13-Octadecadienylacetat Ib.

7. Verfahren nach Anspruch 6, dadurch gekennzeichnet, daß man im Lebensraum der Leopardenmotte (Zeuzera pyrina) ein Gemisch aus E2,Z13-Octadecadienylacetat Ia und Z2,Z13-Octadecadienylacetat Ib in einer solchen Menge ausbringt, daß die männlichen Tiere bei der Auffindung der weiblichen Tiere gestört werden.

8. Mittel zur Bekämpfung der Leopardenmotte (Zeuzera pyrina), enthaltend ein Gemisch aus E2,Z13-Octadecadienylacetat Ia und Z2,Z13-Octadecadienylacetat Ib.

9. Mittel zur Bekämpfung der Leopardenmotte (Zeuzera pyrina) nach Anspruch 8, enthaltend 50 Gew.-% bis 90 Gew.-% an E2,Z13-Octadecadienylacetat Ia.

10. Mittel nach Anspruch 9, enthaltend neben E2,Z13-Octadecadienylacetat Ia und Z2,Z13-Octadecadienylacetat Ib zusätzlich mindestens einen der Alkohole der Formeln VIa und VIb gemäß Anspruch 1.

11. Verwendung von E2,Z13-Octadecadienylacetat Ia zur Bekämpfung der Leopardenmotte (Zeuzera pyrina) mittels der Verwirrungsmethode.

12. Verwendung eines Gemisches aus E2,Z13-Octadecadienylacetat Ia und Z2,Z13-Octadecadienylacetat Ib zur Bekämpfung der Leopardenmotte (Zeuzera pyrina) mittels der Verwirrungsmethode.

13. Verwendung eines Gemisches, enthaltend 50 Gew.-% bis 90 Gew.-% an E2,Z13-Octadecadienylacetat Ia, sowie zusätzlich Z2,Z13-Octadecadienylacetat Ib zur Bekämpfung der Leopardenmotte (Zeuzera pyrina) mittels der Verwirrungsmethode.

14. Verwendung eines Gemisches, enthaltend neben E2,Z13-Octadecadienylacetat Ia und Z2,Z13-Octadecadienylacetat Ib zusätzlich mindestens einen der Alkohole der Formeln VIa und VIb gemäß Anspruch 1 zur Bekämpfung der Leopardenmotte (Zeuzera pyrina) mittels der Verwirrungsmethode.

## Claims

1. A process for the preparation of a mixture of E2,Z13-octadecadienyl acetate Ia and Z2,Z13-octadecadienyl acetate Ib 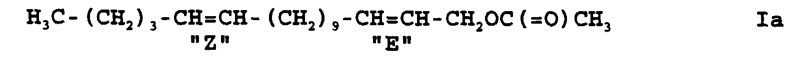 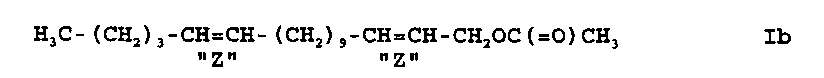 wherein Z11-hexadecenal II 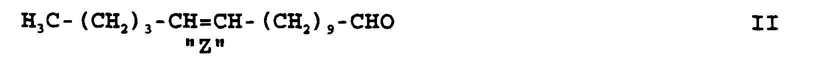 is reacted in a conventional manner in the presence of a base with a phosphonium salt of the formula III
(C₆H₅)₃P⁺CH₂CH(OR¹)OR²X ⁻ III
where R¹ and R² are each alkyl of 1 to 8 carbon atoms or together form -CH₂CH₂- or -CH₂CH₂CH₂-, where in these chains from one to three hydrogen atoms can be replaced by C₁-C₃-alkyl groups, and X is halogen, to give the E2,Z13-octadecadienyl acetal IVa or Z2,Z13-octadecadienyl acetal IVb 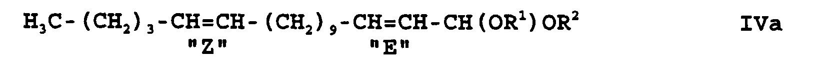 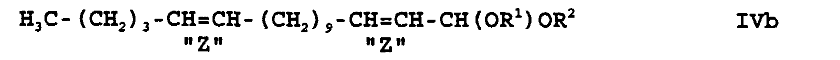 or with simultaneous cleavage of the acetal to give the E2,Z13-octadecadienal Va or Z2,Z13-octadecadienal Vb 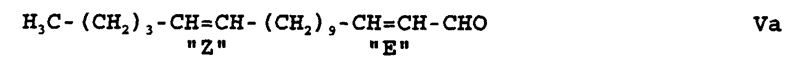 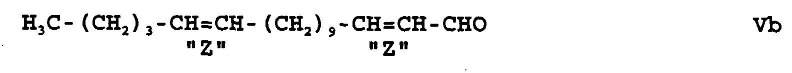 IVa and IVb or Va and Vb are then reduced to the corresponding alcohol VIa or VIb 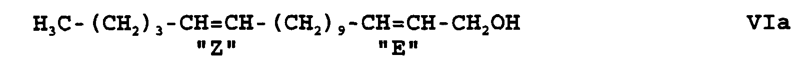 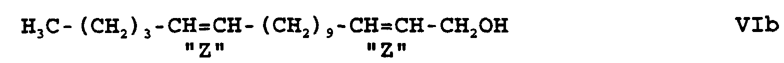 and VIa and VIb are acetylated in a conventional manner.

2. An E2,Z13-octadecadienyl acetal or Z2,Z13-octadecadienyl acetyl of the formula IVa or IVb 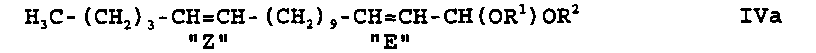 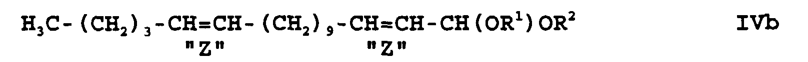 where R¹ and R² are each alkyl of 1 to 8 carbon atoms or together form -CH₂CH₂- or -CH₂CH₂CH₂-, where in these chains from one to three hydrogen atoms may be replaced by C₁-C₃-alkyl groups.

3. Use of an E2,Z13-octadecadienyl acetal or Z2,Z13-octadecadienyl acetal of the formula IVa or IVb as claimed in claim 2 as an intermediate.

4. Use of an E2,Z13-octadecadienyl acetal or a Z2,Z13-octadecadienyl acetal of the formula IVa or IVb as claimed in claim 3 for the preparation of a mixture of E2,Z13-octadecadienyl acetate Ia and Z2,Z13-octadecadienyl acetate Ib.

5. A method for controlling the leopard moth (Zeuzera pyrina) by means of E2,Z13-octadecadienyl acetate Ia.

6. A method for controlling the leopard moth (Zeuzera pyrina) by means of a mixture of E2,Z13-octadecadienyl acetate Ia and 22,Z13-octadecadienyl acetate Ib.

7. A method as claimed in claim 6, wherein a mixture of E2,Z13-octadecadienyl acetate Ia and 22,Z13-octadecadienyl acetate Ib is applied in the habitat of the leopard moth (Zeuzera pyrina) in an amount such that the male animals are disturbed in their attempt to find the female animals.

8. An agent for controlling the leopard moth (Zeuzera pyrina), containing a mixture of E2,Z13-octadecadienyl acetate Ia and Z2,Z13-octadecadienyl acetate Ib.

9. An agent for controlling the leopard moth (Zeuzera pyrina) as claimed in claim 8, containing from 50 to 90% by weight of E2,Z13-octadecadienyl acetate Ia.

10. An agent as claimed in claim 9, containing, in addition to E2,Z13-octadecadienyl acetate Ia and Z2,Z13-octadecadienyl acetate Ib, at least one of the alcohols of the formulae VIa and VIb claimed in claim 1.

11. Use of E2,Z13-octadecadienyl acetate Ia for controlling the leopard moth (Zeuzera pyrina) by means of the confusion method.

12. Use of a mixture of E2,Z13-octadecadienyl acetate Ia and Z2,Z13-octadecadienyl acetate Ib for controlling the leopard moth (Zeuzera pyrina) by means of the confusion method.

13. Use of a mixture containing from 50 to 90 % by weight of E2,Z13-octadecadienyl acetate Ia and in addition Z2,Z13-octadecadienyl acetate Ib for controlling the leopard moth (Zeuzera pyrina) by means of the confusion method.

14. Use of a mixture containing, in addition to E2,Z13-octadecadienyl acetate Ia and Z2,Z13-octadecadienyl acetate Ib, at least one of the alcohols of the formulae VIa and VIb as claimed in claim 1 for controlling the leopard moth (Zeuzera pyrina) by means of the confusion method.

## Revendications

1. Procédé pour la préparation d'un mélange d'acétate de E2,Z13-octadécadiényle Ia et d'acétate de Z2,Z13-octadécadiényle Ib 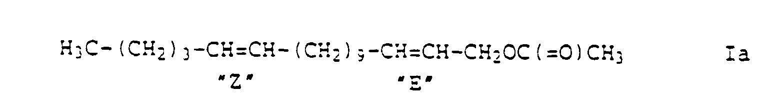 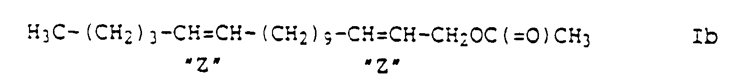 caractérisé par le fait qu'on fait réagir du Z11-hexadécénal II 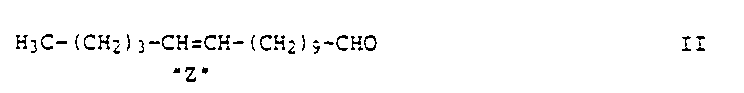 de manière connue en soi en présence d'une base avec un sel de phosphonium de formule III
(C₆H₅)₃P⁺CH₂CH(OR¹)OR² X⁻ III
où R¹ et R² désignent un groupe alkyle ayant 1 à 8 atomes de carbone ou représentent ensemble -CH₂CH₂- ou -CH₂CH₂CH₂-, tandis que dans ces chaînes un à trois atomes d'hydrogène peuvent être remplacés par des groupes alkyle en C₁-C₃, et X désigne un halogène, pour former l'acétal de E2,Z13-octadécadiényle IVa ou l'acétal de Z2,Z13-octadécadiényle IVb respectivement 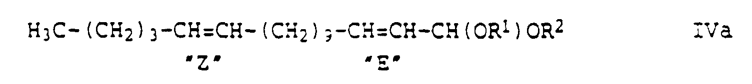 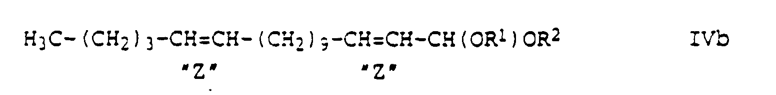 ou avec coupure simultanée de l'acétal pour former le E2,Z13-octadécadiénal Va ou le Z2,Z13-octadécadiénal Vb respectivement 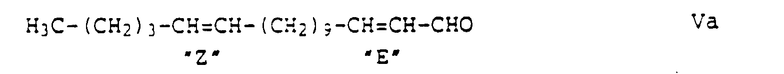 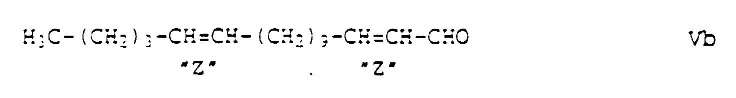 on réduit ensuite IVa et IVb ou respectivement Va et Vb en l'alcool correspondant VIa ou VIb respectivement 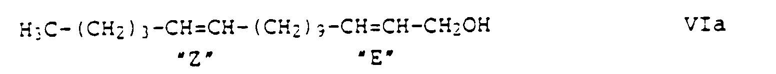 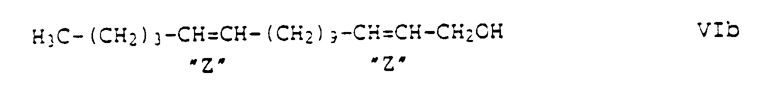 et on acétyle VIa et VIb de manière connue en soi.

2. Acétals de E2,Z13-octadécadiényle et acétals de Z2,Z13-octadécadiényle de formules IVa et IVb 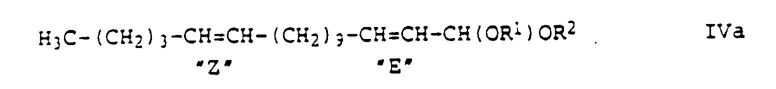 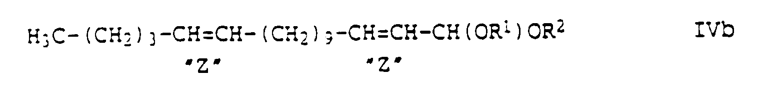 où R¹ et R² désignent un groupe alkyle ayant 1 à 8 atomes de carbone ou représentent ensemble -CH₂CH₂- ou -CH₂CH₂CH₂-, tandis que dans ces chaînes un à trois atomes d'hydrogène peuvent être remplacés par des groupes alkyle en C₁-C₃.

3. Utilisation des acétals de E2,Z13-octadécadiényle et acétals de Z2,Z13-octadécadiényle de formules IVa et IVb selon la revendication 2 comme produits intermédiaires.

4. Utilisation des acétals de E2,Z13-octadécadiényle et acétals de Z2,Z13-octadécadiényle de formules IVa et IVb selon la revendication 3 pour la préparation d'un mélange d'acétate de E2,Z13-octadécadiényle Ia et d'acétate de Z2,Z13-octadécadiényle Ib.

5. Procédé pour la lutte contre la teigne du léopard (Zeuzera pyrina) au moyen d'acétate de E2,Z13-octadécadiényle Ia.

6. Procédé pour la lutte contre la teigne du léopard (Zeuzera pyrina) au moyen d'un mélange d'acétate de E2,Z13-octadécadiényle Ia et d'acétate de Z2,Z13-octadécadiényle Ib.

7. Procédé selon la revendication 6, caractérisé par le fait qu'on met en oeuvre dans le biotope de la teigne du léopard (Zeuzera pyrina) un mélange d'acétate de E2,Z13-octadécadiényle Ia et d'acétate de Z2,Z13-octadécadiényle Ib en une quantité telle que les animaux mâles soient perturbés lors de la recherche des animaux femelles.

8. Moyen pour la lutte contre la teigne du léopard (Zeuzera pyrina), contenant un mélange d'acétate de E2,Z13-octadécadiényle Ia et d'acétate de Z2,Z13-octadécadiényle Ib.

9. Moyen pour la lutte contre la teigne du léopard (Zeuzera pyrina) selon la revendication 8, contenant 50 % en poids à 90 % en poids d'acétate de E2,Z13-octadécadiényle Ia.

10. Moyen selon la revendication 9, contenant, outre l'acétate de E2,Z13-octadécadiényle Ia et l'acétate de Z2,Z13-octadécadiényle Ib, au moins l'un des alcools de formules VIa et VIb selon la revendication 1.

11. Utilisation d'acétate de E2,Z13-octadécadiényle Ia pour la lutte contre la teigne du léopard (Zeuzera pyrina) au moyen de la méthode de l'embrouillement.

12. Utilisation d'un mélange d'acétate de E2,Z13-octadécadiényle Ia et d'acétate de Z2,Z13-octadécadiényle Ib pour la lutte contre la teigne du léopard (Zeuzera pyrina) au moyen de la méthode de l'embrouillement.

13. Utilisation d'un mélange contenant 50 % en poids à 90 % en poids d'acétate de E2,Z13-octadécadiényle Ia, ainsi qu'en outre de l'acétate de Z2,Z13-octadécadiényle Ib, pour la lutte contre la teigne du léopard (Zeuzera pyrina) au moyen de la méthode de l'embrouillement.

14. Utilisation d'un mélange contenant, outre de l'acétate de E2,Z13-octadécadiényle Ia et de l'acétate de Z2,Z13-octadécadiényle Ib, également au moins l'un des alcools de formules VIa et VIb selon la revendication 1 pour la lutte contre la teigne du léopard (Zeuzera pyrina) au moyen de la méthode de l'embrouillement.
